# EUROPEAN PATENT SPECIFICATION

(11) **EP 1 377 560 B1**
(45) Date of publication and mention of the grant of the patent: **21.09.2005**
(21) Application number: 02720172.2
(22) Date of filing: 05.04.2002
(51) Int. Cl.: C07D 295/215, A61K 31/495, A61P 25/00

(54) **PIPERAZINE DERIVATIVES AS TACHYKININ ANTAGONISTS**
PIPERAZINDERIVATIVE ALS TACHYKININ ANTAGONISTEN
DERIVES PIPERAZINES COMME ANTAGONISTES DES TACHYKININES

(30) Priority: 05.04.2001 GB 0108594
(43) Date of publication of application: 07.01.2004
(73) Proprietor: GLAXO GROUP LIMITED, Greenford, Middlesex UB6 ONN (GB)
(72) Inventor: PENTASSUGLIA, Giorgio, c/o GlaxoSmithKline SpA, I-37135 Verona (IT)
(74) Representative: Giddings, Peter John
(86) International application number: PCT/GB2002/001602
(87) International publication number: WO 2002/081461

(56) References cited:
- WO-A-01/25219
- WO-A-97/32865
- WO-A-98/57954

## Description

The present invention relates to piperazine derivatives, to processes for their preparation, to pharmaceutical compositions containing them and to their medical use.

WO 01/25219 describes piperazine derivatives of general formula (A) and pharmaceutically acceptable salts and solvates thereof 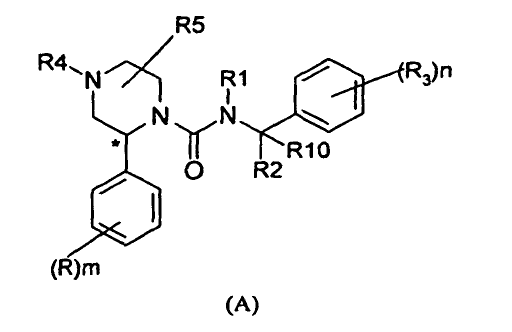 wherein R₄, R₅ and R₁₀ are inter alia hydrogen, R₁ and R₂ are inter alia hydrogen or C₁₋₄ alkyl, R and R₃ are inter alia C₁₋₄ alkyl or halogen. The compounds are antagonists of tachykinins including substance P and other neurokinins.

WO 97/32865 describes piperazine compounds having affinity for neurokinin receptors of formula (B) 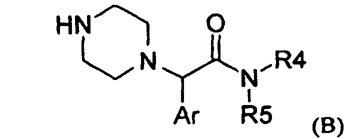 wherein Ar is interalia an optionally substituted phenyl; R₄ is interalia phenyl(C ₁₋₄)alkyl,in which the phenyl group is optionally substituted by 1 to 3 groups selected inter alia from halogen, C ₁₋₄ alkyl, C ₁₋₄ alkyoxy, trifluoromethyl or trifluoromethoxy; R₅ is hydrogen, or C₁₋₄ alkyl.

WO 98/57954 discloses inter alia compounds of formula(C) 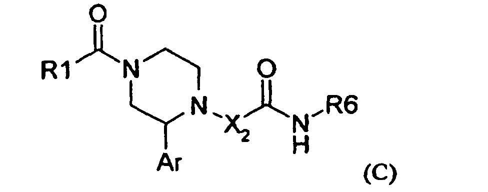 wherein R1 is aryl which may have substituent(s ); aryl is interalia a phenyl which may have substituents; X2 is lower alkylene; R6 is saturated heterocyclic which may have substituent(s) . These compounds and its pharmaceutical acceptable salts are useful for treating or preventing Tachykinin-mediated diseases in human or animals.

Tachykinins are a family of peptides that share a common carboxyl-terminal sequence (Phe-X-Gly-Leu-Met-NH2). They are actively involved in the physiology of both lower and advanced lifeforms. In mammalian lifeforms the main tachykinins are subtance P (SP), Neurokinin A (NKA) and Neurokinin B (NKB) which act as neurotransmitters and neuromodulators. Mammalian tachikynins may contribute to the pathophysiology of a number of human diseases.

Three types of tachykinins receptors have been identified, namely NK1 (SP-preferring), NK2 (NKA-preferring) and NK3 (NKB-preferring) which are widely distributed throughout the central nervous (CNS) and peripheral nervous system.

We have now found that a particular class of compounds, which is not specifically disclosed therein, is particularly useful in the treatment of conditions mediated by tachykinins including substance P and other neurokinins.

We have discovered a novel class of compounds particularly useful for the treatment of depressive states and /or anxiety.

The present invention thus provides compounds of formula (I) 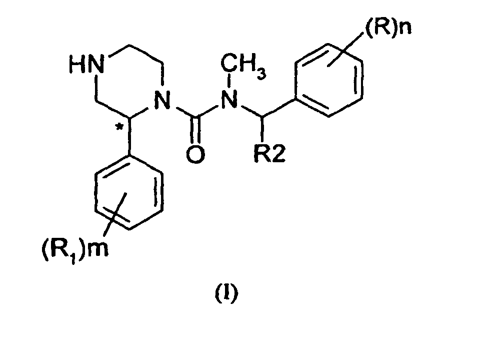 wherein
R and R ₁ represent independently halogen or C₁₋₄ alkyl;
R₂ is hydrogen or C₁₋₄ alkyl;
n and m are independently 1 or 2;
and pharmaceutically acceptable salts and solvates thereof.

Suitable pharmaceutically acceptable salts of the compounds of general formula (I) include acid addition salts formed with pharmaceutically acceptable organic or inorganic acids, for example hydrochlorides, hydrobromides, sulphates, alkyl- or arylsulphonates (e.g. methanesulphonates or p-toluenesulphonates), phosphates, trifluoroacetates, acetates, citrates, succinates, tartrates, fumarates and maleates.

The solvates may, for example, be hydrates.

References hereinafter to a compound according to the invention include both compounds of formula (I) and their pharmaceutically acceptable acid addition salts together with pharmaceutically acceptable solvates.

It will be appreciated by those skilled in the art that the compounds of formula (I) contain at least one chiral centre (namely the carbon atom shown as * in formula (I)) and these may be represented by the formulae (1a) and (1b)

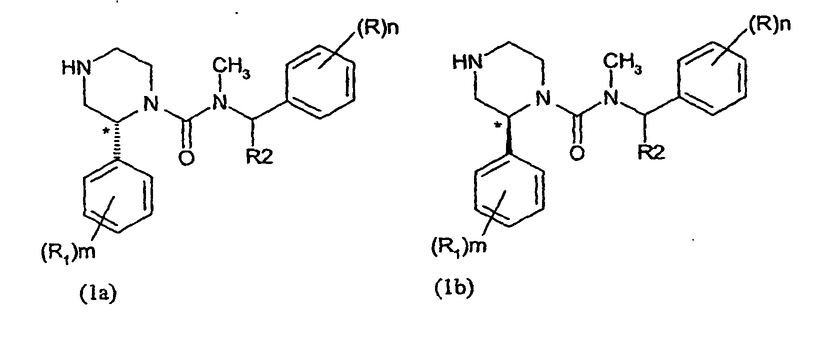

The wedge shaped bond indicates that the bond is above the plane of the paper. The broken bond indicates that the bond is below the plane of the paper.
The configuration shown for the chiral carbon atom indicated as * in formula la is α and in formula 1b is β.
In general, in the specific compounds named below, the β configuration at the chiral carbon atom indicated as * corresponds to the S isomer and the α configuration corresponds to the R isomer.

The assignment of the R and S configuration of the asymmetric carbon atoms of the compounds of the invention has been made according to the rules of Cahn, Ingold and Prelog 1956,12,81.

It is to be understood that all stereoisomeric forms, including all enantiomers and mixtures thereof, are encompassed within the scope of the present invention and the reference to compound of formula(I) include all stereoisomeric forms unless otherwise stated.

The term C₁₋₄ alkyl refers to a straight or branched alkyl group containing from 1 to 4 carbon atoms; examples of such groups include methyl, ethyl, propyl, isopropyl, n-butyl, isobutyl or tert butyl.

The term halogen refers to a fluorine, chlorine, bromine or iodine atom.

When R represents halogen this is suitably chlorine or fluorine and when R is C₁₋₄ alkyl this is suitably methyl.

When either m or n is 2, the group R and R₁ respectively may be the same or different.

Suitable values for R₁ include fluorine or methyl.

Suitable values for R₂ include hydrogen or methyl.

A preferred class of compounds of formula (I) are those wherein the chiral carbon atom indicated as * is in β configuration.

For compounds of formula (I) n is conveniently 2.

For compounds of formula (I) m is conveniently 2 and within these compounds those wherein R₁ is at the 2 and the 4 position of the phenyl ring are preferred.

A preferred group of compounds of formula (I) are those wherein R is selected from fluorine, chlorine or methyl, R₁ is fluorine or methyl at the 2 and the 4 position of the phenyl ring, R₂ is hydrogen or methyl, n and m are 2.

Preferred compounds according to the invention are:
2-(S)-(4-Fluoro-2-methyl-phenyl)-piperazine-1-carboxylic acid (2,4-dimethyl-benzyl)-methylamide acetate;
2-(S)-(4-Fluoro-2-methyl-phenyl) piperazine-1-carboxylic acid, (3,5-dimethyl-benzyl)-methylamide;
2-(S)-(4-Fluoro-2-methyl-phenyl)-piperazine-1-carboxylic acid, (3,5-dichloro-benzyl)-methylamide;
2-(S)-(4-Fluoro-2-methyl-phenyl)-piperazine-1-carboxylic acid, (3-chloro-4-fluoro-benzyl)-methylamide;
2-(S)-(4-Fluoro-2-methyl-phenyl)-piperazine-1-carboxylic acid, (4-fluoro-benzyl)-methylamide;
2-(S)-(4-Fluoro-2-methyl-phenyl)-piperazine-1-carboxylic acid, (3,5-difluoro-benzyl)-methylamide;
2-(S)-(4-Fluoro-2-methyl-phenyl)-piperazine-1-carboxylic acid, (2,5-dichloro-benzyl)-methylamide;
2-(S)-(4-fluoro-2-methyl-phenyl)-piperazine-1-carboxylic acid, (3,4-dichlorobenzyl)-methylamide;
and pharmaceutically acceptable salts (e.g. hydrochloride, acetate and trifluoroacetate) or solvates thereof.

A further preferred compound of the invention is:
2-(S)-(4-Fluoro-2-methyl-phenyl)-piperazine-1-carboxylic acid, (2,5-dichloro-benzyl)-methylamide
and pharmaceutically acceptable salts (e.g. hydrochloride) and solvates thereof.

The compounds of the invention are antagonists of tachykinins, including substance P and other neurokinins, both in vitro and in vivo and are thus of use in the treatment of conditions mediated by tachykinins, including substance P and other neurokinins.

The compounds of the present invention have also activity as serotonin reuptake inhibitors.

Thus the compounds of the present invention combine dual activity as tachykinins antagonists, including substance P and other neurokinins, and as serotonin reuptake inhibitors.

NK₁-receptor binding affinity has been determined in vitro by the compounds' ability to displace [3H] - substance P (SP) from recombinant human NK₁ receptors expressed in Chinese Hamster Ovary (CHO) cell membranes.
CHO cell membranes were prepared by using a modification of the method described by Dam T and Quirion R (Peptides, 7:855-864, 1986). Thus ligand binding was performed in 0.4 ml of 50 mM HEPES, pH 7.4, containing 3 mM MnCl₂, 0.02% BSA, 0.5 nM [³H]-Substance P (30ö56 Ci/mmol, Amersham), a final membrane concentration of 25 µg of protein/ml, and the test compounds. The incubation proceeded at room temperature for 40 min. Non-specific binding was determined using excess of Substance P (1 µM) and represents about 6% of the total binding.

Compounds of the invention were further characterised in a functional assay for the determination of their inhibitory effect. Human-NK₁-CHO cells were stimulated with Substance P and the receptor activation was evaluated by measuring the accumulation of cytidinediphosphodiacylglycerol (CDP-DAG), which is the liponucleotide precursor of phosphatidylinositol diphosphate. CDP-DAG accumulates in the presence of Li⁺ as a consequence of the receptor mediated activation of phospholipase C (PLC) (Godfrey, Biochem. J., 258:621-624, 1989). The method is described in detail by Ferraguti et al. (Mol. Cell. Neurosci., 5:269-276, 1994).

The action of the compounds of the invention at the NK₁ receptor and/or serotonin transporter may be determined by using conventional animal models. Thus the ability to bind at the NK₁ receptor and/or serotonin transporter was determined using the guinea pig pup isolation calls model as described by Pettijohn, Psychol. Rep., 1979 and Rupniak et al., Neuropharmacology, 2000.

Human Serotonin Transporter (hSERT) binding affinity has been determined in vitro by the compounds' ability to displace [³H]- Imipramine from human serotonin transporter expressed in Human Embryonic Kidney HEK293 cell membranes (Receptor Biology Inc.). For the binding reaction, 4 nM of [³H]- Imipramine (703 GBq/mmol, Amersham) were incubated with 0.02 mg/ml of cell membrane and the compound to be tested at different concentrations (7 concentration points) in 50 mM Tris HCl, pH 7.5, 120 mM of NaCl and 5 mM KCl. The reaction was performed for 60 min at 4°C and was terminated by filtration through GF/B Unifilters 96 wells/case (presoaked in 0.5 % PEI) using a Cell Harvester (Packard), Scintillation fluid was added to each filtered spot and radioactivity was determined using a scintillation counter (TopCount (Packard)). Non-specific binding was determined using Imipramine (100µM) and represents about 5% of the total binding. Competition experiments were conducted with duplicate determination for each point. Msat601 software package was used to elaborate the competition binding data. IC₅₀ values were converted to Kᵢ values using Cheng-Prusoff equation.

The inhibitory activity of the compounds at the rat serotonin transporter has been determined in vitro using rSERT-LLCPK cells (LLCPK cells tranfected with the rat SERT). The cells have been plated onto 96-well plates (60000 cells/well). After 24 hr, cells have been washed in uptake buffer (Hank's balanced salt solution + 20 mM Hepes) and pre-incubated for 10 min at RT with 50 µl of buffer containing the test compounds. 50 µl of 50 nM [3H] Serotonin (5HT) solution (final concentration: 25 nM [3H] 5HT) have been added and plates have been incubated for 7 min at RT, during which cells take up radiolabelled 5HT. Aspirating the solution and rapidly washing the cells with cold buffer has terminated the uptake.
The amount of radioactive 5HT incorporated in the cells has been then measured by adding the scintillation cocktail directly onto the cells and reading the plate in the Top Count. The data have been digitally processed to obtain the pIC50 values of the antagonists. The pKi values have been calculated using the Chen-Prusoff equation.

Compounds of the invention are useful in the treatment of CNS disorders and psychotic disorders, in particular in the treatment or prevention of depressive states and /or in the treatment of anxiety.
Depressive states include major depressive disorders including bipolar depression, unipolar depression, single or recurrent major depressive episodes with or without psychotic features, catatonic features, melancholic features, atypical features or postpartum onset, the treatment of anxiety and the treatment of panic disorders. Other mood disorders encompassed within the term major depressive disorders include dysthymic disorder with early or late onset and with or without atypical features, neurotic depression, post traumatic stress disorders and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.

Compounds of the invention are useful as analgesics. In particular they are useful in the treatment of traumatic pain such as postoperative pain; traumatic avulsion pain such as brachial plexus; chronic pain such as arthritic pain such as occurring in osteo-, rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy, phantom limb pain; various forms of headache such as migraine, acute or chronic tension headache, temporomandibular pain, maxillary sinus pain, cluster headache; odontalgia; cancer pain; pain of visceral origin; gastrointestinal pain; nerve entrapment pain; sport's injury pain; dysmennorrhoea; menstrual pain; meningitis; arachnoiditis; musculoskeletal pain; low back pain e.g. spinal stenosis; prolapsed disc; sciatica; angina; ankylosing spondyolitis; gout; burns; scar pain; itch; and thalamic pain such as post stroke thalamic pain.

Compounds of the invention are also useful in the treatment of sleep disorders including dysomnia, insomnia, sleep apnea, narcolepsy, and circadian ritmic disorders.

Compounds of the invention are also useful in the treatment or prevention of the cognitive disorders. Cognitive disorders include dementia, amnestic disorders and cognitive disorders not otherwise specified.

Furthermore compounds of the invention are also useful as memory and/or cognition enhancers in healthy humans with no cognitive and/or memory deficit.

Compounds of the invention are also useful in the treatment of tolerance to and dependence on a number of substances. For example, they are useful in the treatment of dependence on nicotine, alcohol, caffeine, phencyclidine (phencyclidine like compounds), or in the treatment of tolerance to and dependence on opiates (e.g. cannabis, heroin, morphine) or benzodiazepines; in the treatment of cocaine, sedative ipnotic, amphetamine or amphetamine-related drugs (e.g. dextroamphetamine, methylamphetamine) addiction or a combination thereof.

Compounds of the invention are also useful as anti-inflammatory agents. In particular they are useful in the treatment of inflammation in asthma, influenza, chronic bronchitis and rheumatoid arthritis; in the treatment of inflammatory diseases of the gastrointestinal tract such as Crohn's disease, ulcerative colitis, inflammatory bowel disease and non-steroidal anti-inflammatory drug induced damage; inflammatory diseases of the skin such as herpes and eczema; inflammatory diseases of the bladder such as cystitis and urge incontinence; and eye and dental inflammation.

Compounds of the invention are also useful in the treatment of allergic disorders, in particular allergic disorders of the skin such as urticaria, and allergic disorders of the airways such as rhinitis.

Compounds of the invention are also useful in the treatment of emesis, i.e. nausea, retching and vomiting. Emesis includes acute emesis, delayed emesis and anticipatory emesis. The compounds of the invention are useful in the treatment of emesis however induced. For example, emesis may be induced by drugs such as cancer chemotherapeutic agents such as alkylating agents, e.g. cyclophosphamide, carmustine, lomustine and chlorambucil; cytotoxic antibiotics, e.g. dactinomycin, doxorubicin, mitomycin-C and bleomycin; anti-metabolites, e.g. cytarabine, methotrexate and 5- fluorouracil; vinca alkaloids, e.g. etoposide, vinblastine and vincristine; and others such as cisplatin, dacarbazine, procarbazine and hydroxyurea; and combinations thereof; radiation sickness; radiation therapy, e.g. irradiation of the thorax or abdomen, such as in the treatment of cancer; poisons; toxins such as toxins caused by metabolic disorders or by infection, e.g. gastritis, or released during bacterial or viral gastrointestinal infection; pregnancy; vestibular disorders, such as motion sickness, vertigo, dizziness and Meniere's disease; post-operative sickness; gastrointestinal obstruction; reduced gastrointestinal motility; visceral pain, e.g. myocardial infarction or peritonitis; migraine; increased intercranial pressure; decreased intercranial pressure (e.g. altitude sickness); opioid analgesics, such as morphine; and gastro-oesophageal reflux disease, acid indigestion, over-indulgence of food or drink, acid stomach, sour stomach, waterbrash/regurgitation, heartburn, such as episodic heartburn, nocturnal heartburn, and meal-induced heartburn and dyspepsia.

Compounds of the invention are also useful in the treatment of gastrointestinal disorders such as irritable bowel syndrome; skin disorders such as psoriasis, pruritis and sunburn; vasospastic diseases such as angina, vascular headache and Reynaud's disease; cerebral ischeamia such as cerebral vasospasm following subarachnoid haemorrhage; fibrosing and collagen diseases such as scleroderma and eosinophilic fascioliasis; disorders related to immune enhancement or suppression such as systemic lupus erythematosus and rheumatic diseases such as fibrositis; and cough.

Compounds of the invention have been found to exhibit anxiolytic activity in conventional tests. For example in marmoset human threat test (Costall et al.; 1988).

The invention therefore provides a compound of formula (I) or a pharmaceutically acceptable salt or solvate thereof for use in therapy, in particular in human medicine.

There is also provided as a further aspect of the invention the use of a compound of formula (I) or a pharmaceutically acceptable salt or solvate thereof in the preparation of a medicament for use in the treatment of conditions mediated by tachykinins (including substance P and other neurokinins).

According to a further or alternative aspect, the present invention provides the use of a compound of formula (I) or a pharmaceutically acceptable salt or solvate thereof in the preparation of a medicament for use in the treatment of depressive states and/or anxiety.

The compounds of formula I can be used in a method for the treatment of a mammal, including man, in particular in the treatment of conditions mediated by tachykinins, including substance P and other neurokinins.

A method for the treatment of depressive states and/or anxiety comprises the administration of an effective amount of a compound combining dual activity as tachykinins antagonists, including substance P and other neurokinins, and as serotonin reuptake inhibitors.

In a further aspect of the present invention there is provided the use of a compound combining dual activity as tachykinins antagonists (including substance P and other neurokinins) and as serotonin reuptake inhibitors in the preparation of a medicament for use in the treatment of depression and/or anxiety.

A compound combining dual activity as tachykinins antagonists (including substance P and other neurokinins) and as serotonin reuptake inhibitors can be used in the treatment of depression and/or anxiety.

It will be appreciated that reference to treatment is intended to include prophylaxis as well as the alleviation of established symptoms.
Compounds of formula (I) may be administered as the raw chemical but the active ingredient is preferably presented as a pharmaceutical formulation.

Accordingly, the invention also provides a pharmaceutical composition which comprises at least one compound of formula (I) or a pharmaceutically acceptable salt thereof and formulated for administration by any convenient route. Such compositions are preferably in a form adapted for use in medicine, in particular human medicine, and can conveniently be formulated in a conventional manner using one or more pharmaceutically acceptable carriers or excipients.

Thus compounds of formula (I) may be formulated for oral, buccal, parenteral, topical (including ophthalmic and nasal), depot or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

A proposed dose of the compounds of the invention is 1 to about 1000mg per day. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.
Thus for parenteral administration a daily dose will typically be in the range of 1 to about 100 mg, preferably 1 to 80 mg per day. For oral administration a daily dose will typically be within the range 1 to 300 mg e.g. 1 to 100 mg.

Compounds of formula (I), and salts and solvates thereof, may be prepared by the general methods outlined hereinafter. In the following description, the groups R, R₁ m and n have the meaning as previously defined for compounds of formula (I) unless otherwise stated.

According to general process (A), a compound of formula (I) may be prepared by reduction of a ketopiperazine of formula (II), wherein Rₐ is hydrogen or a suitable nitrogen protecting group or followed where necessary or desired by removal of any protecting group.

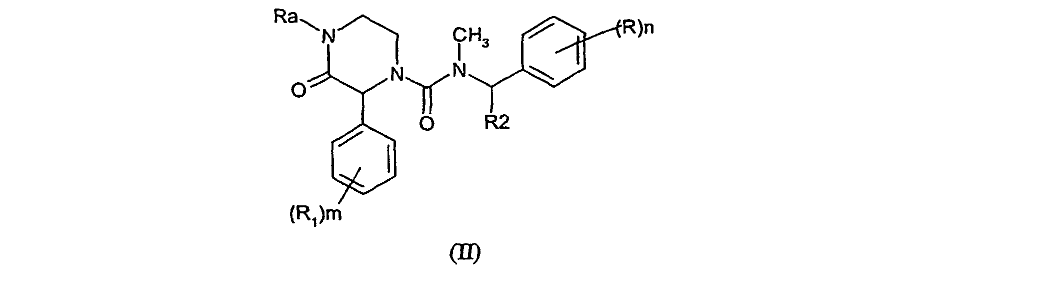

The reaction may be carried out using a suitable metal reducing agent such as a metal hydride, for example a borane hydride, or a metal hydride complex like lithium aluminum hydride, borohydride, or an organo-metallic complex such as borane- methyl sulphide, 9-borabicyclononane (9-BBN), triethylsilane, sodium triacetoxyborohydride, sodium cyanoborohydride.
Alternatively boranes may be produced in situ by reacting Sodium Borohydride in the presence of Iodine, an inorganic acid (e.g. sulphoric acid) or an organic acid such as formic acid, trifluoroacetic, acetic acid, or methansulphonic acid.
Suitable solvents for this reaction are ether (e.g. tetrahydrofuran), or halohydrocarbon (e.g. dichloromethane) or an amide (e.g. N,N-dimethylformamide) at a temperature within the range of room temperature to the reflux temperature of the reaction mixture.

Compounds of formula (II) may be prepared by treating compounds of formula (III) wherein Rₐ has the meaning defined in formula (II) 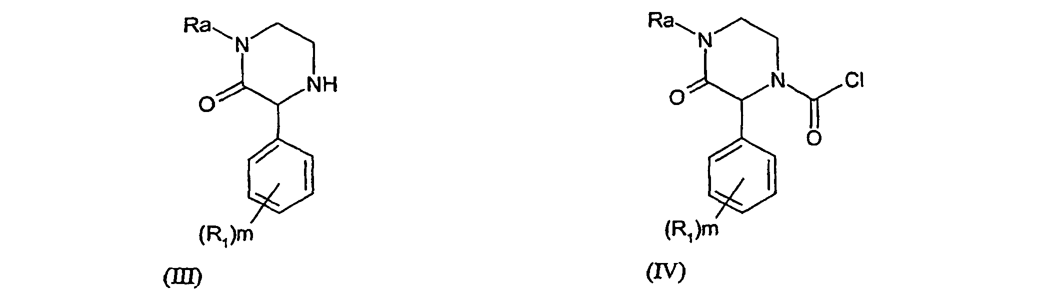 with triphosgene in aprotic solvent such as dichloromethane and in the presence of an organic base such triethylamine to form the intermediate carbonyl chloride compound (IV) which may be isolated if required, followed by reaction of compound (IV) with the amine compound (V)

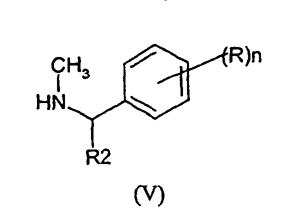

The reaction conveniently takes place in an aprotic solvent such as a hydrocarbon, a halohydrocarbon such as dichloromethane or an ether such as tetrahydrofuran optionally in the presence of a base such as a tertiary amine e.g. diisopropyl ethyl amine.

Compounds of formula (III) may be prepared by reduction of a dihydropyrazin-2-one (VI) using a suitable metal reducing agent such as sodium borohydride. Alternatively, catalytic hydrogenation may be used, for example using Palladium on Carbon catalyst in a suitable solvent such as methanol.

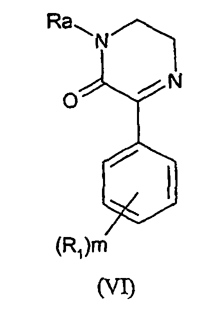

Alternatively compounds of formula (III) may be obtained by reaction of a compound of formula (VII) wherein R₃ is a C₁₋₄ alkyl group and X is a suitable leaving group such as halogen, i.e. bromine or iodine atom, or OSO₂CF₃, 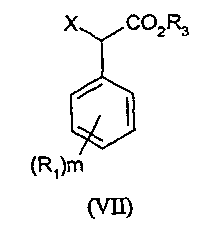 with ethylenediamine. The reaction conveniently takes place in a suitable solvent such as alcohol (i.e. ethanol) at an elevated temperature.

According to a further general process (B) a compound of formula (I) may be prepared by reacting a compound of formula (VIII) 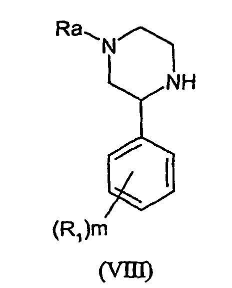 wherein Rₐ represents a nitrogen protecting group, with triphosgene in an aprotic solvent such as dichloromethane or alkyl esters (e.g. ethylacetate) and in the presence of an organic base such triethylamine to form the intermediate carbonyl chloride compound (IVa) which may be isolated if required, followed by reaction of compound (IV) with the amine compound (V)

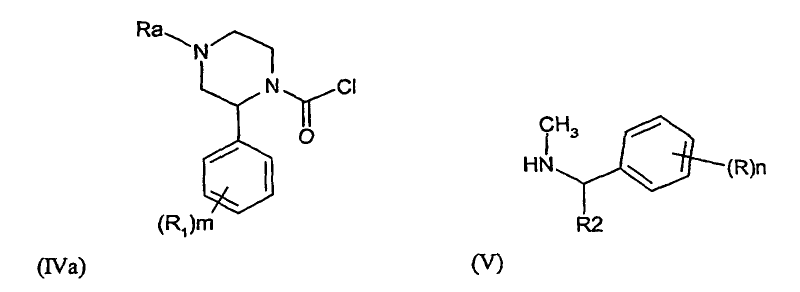

The reaction conveniently takes place in an aprotic solvent, such as a hydrocarbon, a halohydrocarbon such as dichloromethane or an ether such as tetrahydrofuran or alkyl esters (i.e. ethylacetate) optionally in the presence of a base such as a tertiary amine e.g. diisopropyl ethyl amine or triethylamine followed where necessary by deprotection.

Compounds of the invention may be prepared according to the above-described reactions using well-known solid phase synthetic procedures.
Thus, intermediate (VIII) wherein Rₐ is hydrogen may be anchorated to a functionalized Merrifield resin in a suitable solvent such as N,N-dimethylformamide. The functionalized Merrifield resin may be prepared by conventional techniques such as those described in Leger R, Tetrahedron Letters, 1998, vol. 39, 4171-4174.
The cleavage of the resin to obtain compounds of the invention was carried out using conventional methods such as, for example, with an appropriate acid.

When Rₐ is a nitrogen protecting group, examples of suitable groups include alkoxycarbonyl e.g. t-butoxycarbonyl, benzyloxycarbonyl, arylsulphonyl e.g. phenysulphonyl or 2-trimethylsilylethoxymethyl.

Protection and deprotection may be effected using conventional techniques such as those described in "Protective Groups in Organic Synthesis 2^{nd} Ed." by T.W. Greene and P. G. M. Wuts (John Wiley and Sons, 1991) and as described in the examples hereinafter.

Compounds of formula (V), (VI) and (VII) may be prepared by analogous methods to those used for known compounds.

Thus for example compounds of formula (VIII) may be obtained by reduction of a compound of formula (III).
The reaction may be carried out using a suitable metal reducing agent such as a metal hydride, for example a borane hydride, or a metal hydride complex like lithium aluminum hydride, borohydride, or an organo-metallic complex such as borane- methyl sulphide, 9-borabicyclononane (9-BBN), triethylsilane, sodium triacetoxyborohydride, sodium cyanoborohydride.
Alternatively boranes may be produced in situ by reacting Sodium Borohydride in the presence of Iodine, an inorganic acid (e.g. sulphoric) or an organic acid such as formic acid, trifluoroacetic, acetic acid or methansuphonic acid.

Pharmaceutically acceptable salts may also be prepared from other salts, including other pharmaceutically acceptable salts, of the compound of formula (I) using conventional methods.

The compounds of formula (I) may be readily isolated in association with solvent molecules by crystallisation from or evaporation of an appropriate solvent to give the corresponding solvates.

When a specific enantiomer of a compound of general formula (I) is required, this may be obtained for example by resolution of a corresponding enantiomeric mixture of a compound of formula (I) using conventional methods.
Thus specific enantiomers of the compounds of formula (I) may be prepared by reaction of a suitable chiral alcohol, in the presence of a source of a carbonyl group (such as triphosgene or carbonyl diimidazole) separating the resulting diastereoisomeric carbamates by conventional means e.g. chromatography or by fractional crystallisation. The required enantiomer of a compound of general formula (I) may be isolated by removal of carbamate and conversion into the required free base or salts thereof.
Suitable chiral alcohols for use in the process include (*R*)-*sec*-phenylethyl alcohol, etc.

Alternatively, enantiomers of a compound of general formula (I) may be synthesised from the appropriate optically active intermediates using any of the general processes described herein.
Thus for example the required enantiomer may be prepared by the corresponding enantiomeric amine of formula (III) using any of the processes described above for preparing compounds of formula (I) from the amine (III). The enantiomer of amine (III) may be prepared from the racemic amine (III) using conventional procedures such as salt formation with a suitable optically active acid such as L-(+)-mandelic acid or (1S)-(+)-10-camphorsulfonic acid.

In one preferred embodiment of the invention the specific enantiomer of piperazine (IIIa) 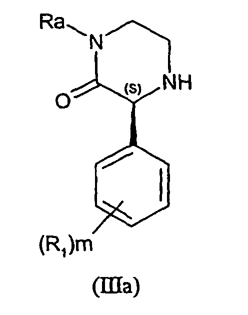 may be prepared by dynamic kinetic resolution of piperazine (III) with a suitable optically active acid such as L-(+)-mandelic acid or (1S)-(+)-10-camphorsulfonic acid in the presence of aromatic aldehyde such as 3,5 dichlorosalicylaldehyde, salicylaldehyde, benzaldehyde, p-nitro benzaldehyde.
A particular preferred aldehyde for use in this reaction is 3,5 dichlorosalicylaldehyde. The reaction is conveniently carried out in an aprotic solvent such as tetrahydrofuran, ethyl acetate at a temperature ranging between 20-60°C.

The invention is further illustrated by the following Intermediates and Examples which are not intended as a limitation of the invention.

In the Intermediates and Examples unless otherwise stated:
Melting points (m.p.) were determined on a Gallenkamp m.p. apparatus or a Büchi 530 melting point apparatus and are uncorrected. All temperatures refers to °C. Infrared spectra were measured on a FT-IR instrument. ¹H-NMR spectra were recorded at 400 or 500 MHz, chemical shifts are reported in ppm (δ) using the residual solvent line as internal standard, while ¹⁹F-NMR were recorded at 400 or 500 MHz using trifluoroacetic acid (-78.5 ppm) as internal standard. MAS-NMR were recorded at 400 MHz using CDC12 as internal standard. The signals are assigned as singlets (s), doublets (d), doublets of doublets (dd), triplets (t), quartets (q) or multiplets (m). Flash column chromatography was carried out over silica gel (Merck AG Darmstaadt, Germany). The following abbreviations are used in the text: AcOEt = ethyl acetate, CH = cyclohexane, DCM = dichloromethane, Et₂O = diethyl ether, DMF = N,N'-dimethylformamide, DIPEA=N,N-diisopropylethylamine MeOH = methanol, TEA = triethylamine, TFA = trifluoroacetic acid, THF = tetrahydrofuran. Tlc refers to thin layer chromatography on silica plates, and dried refers to solution dried over anhydrous sodium sulphate; r.t. (RT) refers to room temperature.

### Intermediate 1

### 2-(S)-(4-Fluoro-2-methyl-phenyl)-3-oxo-piperazine-1-carboxylic acid, (2,4-dimethylbenzyl)-methylamide

TEA (201 µL) and a solution of triphosgene (71 mg) in anhydrous DCM (4.6 mL) were added to a solution of 3-(S)-(4-fluoro-2-methyl-phenyl)-piperazin-2-one (100 mg) in anhydrous DCM (5.0 mL), previously cooled to 0°C under a nitrogen atmosphere. The reaction mixture was stirred from 0°C to r.t. for 4 h.
Then, DIPEA (84 µL) and N-methyl-2,4-dimethyl-benzylamine hydrochloride (105 mg) were added and the reaction mixture was stirred at r.t. for 18h. It was then diluted with further DCM and washed with 1N hydrochloric acid solution. The organic layer was dried and concentrated *in vacuo* to a residue which was purified by flash chromatography (AcOEt/MeOH 95:5) to give the title compound (93 mg) as a yellow foam.
IR (nujol, cm⁻¹): 3195 (NH), 1674 (C=O), 1647 (C=O and C=C).
¹H-NMR (CDCl₃): δ (ppm) 7.24 (dd, 1H), 6.97 (s, 1H), 6.94 (d, 1H), 6.91 (dd, 1H), 6.87 (d, 1H), 6.84 (td, 1H), 6.34 (s, 1H), 5.67 (s, 1H), 4.59 (d, 1H), 4.17, d, 1H), 3.50 (m, 2H), 3.43-3.38 (m, 2H), 2.77 (s, 3H), 2.41 (s, 3H), 2.30 (s, 3H), 2.15 (s, 3H).
MS (ES/+): m/z=384 [MH]⁺.

### Intermediate 2

### 2-(S)-(4-Fluoro-2-methyl-phenyl)-3-oxo-piperazine-1-carboxylic acid, (3,5-dimethylbenzyl) methylamide

TEA (201 µL) and a solution of triphosgene (71 mg) dissolved in anhydrous DCM (5 mL) were added to a solution of 3-(S)-(4-fluoro-2-methyl-phenyl)-piperazin-2-one (100 mg) in anhydrous DCM (5 mL), previously cooled to 0°C under a nitrogen atmosphere. The reaction mixture was stirred for 4 h and the temperature was allowed to reach 23°C. Then, DIPEA (167 µL) and N-methyl-3,5-dimethyl-benzylamine hydrochloride (107 mg) were added. The mixture was stirred at room temperature for 20 h, then a saturated solution of ammonium chloride was added. The organic phase was extracted with DCM, dried and concentrated *in vacuo* to a residue which was purified by flash chromatography (from AcOEt 100% to AcOEt/MeOH 9:1) to give the title compound (118 mg) as a white foam.
IR (nujol, cm⁻¹): 3230 (NH), 1677 (C=O), 1620 (C=C).
¹H-NMR (d₆-DMSO): δ (ppm) 8.11 (bs, 1H); 7.21 (dd, 1H); 6.98 (dd, 1H); 6.90 (m, 1H); 6.81(s, 1H); 6.55 (s, 2H); 5.24 (s, 1H); 4.29 (d, 1H); 4.19 (d, 1H); 3.40 (m, 2H); 3.24 (m, 2H); 2.64 (s, 3H); 2.31 (s, 3 H); 2.14 (s, 6H).
MS (ES/+): m/z=384 [MH]⁺.

### Intermediate 3

### 2-(S)-(4-Fluoro-2-methyl-phenyl)-3-oxo-piperazine-1-carboxylic acid, (3,5-dichlorobenzyl)-methylamide

TEA (219 µL) and a solution of triphosgene (70 mg) in anhydrous DCM (5.0 mL) were added to a solution of 3-(S)-(4-fluoro-2-methyl-phenyl)-piperazin-2-one (109 mg, 0.52 mmol) in anhydrous DCM (5.4 mL) previously cooled at 0°C under a nitrogen atmosphere. The reaction mixture was stirred from 0°C to r.t. for 4 h.
Then, DIPEA (91 µL) and N-methyl-3,5-dichloro-benzylamine hydrochloride (143 mg) were added and the reaction mixture was stirred at r.t. for 18 h. It was then diluted with DCM and washed with 1N hydrochloric acid solution. The organic layer was dried and concentrated *in vacuo* to a residue which was purified by flash chromatography (AcOEt/MeOH 95:5) to give the title compound (147 mg) as a yellow solid.
¹H-NMR (DMSO-d₆): δ (ppm) 8.14 (bs, 1H), 7.44 (t, 1H), 7.22 (dd, 1H), 7.01 (d, 2H), 6.98 (dd, 1H), 6.90 (dt, 1H), 5.26 (s, 1H), 4.33 (AB, 1H), 3.5-3.4 (m, 2H), 3.32 (m, 1H), 3.22 (m, 1H), 2.72, (s, 3H), 2.32 (s, 3H).
MS (FAB⁺/NBA): m/z=424 [MH]⁺.

### Intermediate 4

### 4-[(3-Chloro-4-fluoro-benzyl)-methyl-carbamoyl]-3-(S)-(4-fluoro-2-methyl-phenyl)-piperazine-1-carboxylic acid, benzyl ester

TEA (129 µL) and a solution of triphosgene (41 mg) in anhydrous DCM (3.0 mL) were added to a solution of 1-(benzyloxycarbonyl)-3-(S)-(4-fluoro-2-methyl-phenyl)-piperazine (101 mg, 0.31 mmol) in anhydrous DCM (3.2 mL) previously cooled to 0°C under a nitrogen atmosphere. The reaction mixture was stirred from 0°C to r.t. for 4 hr.
Then, DIPEA (54 µL) and N-methyl-3-chloro-2-fluoro-benzylamine hydrochloride (90 mg) were added and the reaction mixture was stirred at r.t. for 18 h. It was then diluted with further DCM and washed with 1N hydrochloric acid solution. The organic layer was dried and concentrated *in vacuo* to a residue which was purified by flash chromatography (CH/AcOEt 6:4) to give the title compound (138 mg) as a white foam.
¹H-NMR (DMSO-d₆): δ (ppm) 7.34-7.24 (m, 7H), 7.06 (d, 1H), 7.03-6.97 (m, 2H), 6.91 (t, 1H), 5.09 (d, 2H), 4.40 (bs, 1H), 4.39 (d, 1H), 4.24 (d, 1H), 3.79 (m, 2H), 3.44-3.30 (m, 2H), 3.09 (bs, 1H), 2.94 (bs, 1H), 2.93 (bs, 3H), 2.87 (s, 3H).
MS (FAB⁺/NBA): m/z=528 [MH]⁺.

### Intermediate 5

### 4-[(4-Fluoro-benzyl)-methyl-carbamoyl]-3-(S)-(4-fluoro-2-methyl-phenyl)-piperazine-1-carboxylic acid, benzyl ester

TEA (77 µL) and a solution of triphosgene (24 mg) in anhydrous DCM (1.8 mL) was added to a solution of 1-(benzyloxycarbonyl)-3-(S)-(4-fluoro-2-methyl-phenyl)-piperazine (60 mg) in anhydrous DCM (1.8 mL) previously cooled to 0°C under a nitrogen atmosphere. The reaction mixture was stirred from 0°C to r.t. for 4 h.
Then, N-methyl-4-fluoro-benzylamine hydrochloride (35 mg) was added. The reaction mixture was stirred at r.t. for 18 h. It was then diluted with further DCM and washed with 1N hydrochloric acid solution. The organic layer was dried, concentrated *in vacuo* and the residue was purified by flash chromatography (CH/AcOEt 6:4) to give the title compound (136 mg) as a colourless oil.
IR (film, cm⁻¹): 1701 (C=O), 1660 (C=O urea).
¹H-NMR (DMSO-d₆): δ (ppm) 7.40-7.30 (m, 5H), 7.30 (dd, 1H), 7.10-7.00 (m, 4H), 6.97 (dd, 1H), 6.91 (td, 1H), 5.10 (dd, 2H), 4.48 (m, 1H), 4.32 (dd, 2H), 3.80 (m, 2H), 3.42 (m, 2H), 3.14 (m, 1H), 2.99 (m, 1H), 2.79 (s, 3H), 2.31 (s, 3H).
MS (ES/+): m/z=494 [MH]⁺.

### Intermediate 6

### Polymer-bound 2-(S)-(4-fluoro-2-methyl-phenyl)-piperazine

p-Nitrophenyl chloroformate (0.363 g) was dissolved in DCM (3 mL) and added to the Boc-like resin (0.3 g, 1 mmol/g) prepared following literature procedure (Leger R, Tetrahedron Letters, 1998, vol 39, 4171-4174) under a nitrogen atmosphere. Then, N-methylmorpholine (0.198 mL) was added and the reaction mixture was shaken at r.t. overnight. The solution was drained and the resin was washed with THF and DCM under a nitrogen atmosphere, then dried to give the p-nitrophenylcarbonate resin.
The activated resin (0.2 g) was swollen in DMF (2.5 mL) and a solution of 2-(S)-(4-fluoro-2-methyl-phenyl)-piperazine dihydrochloride (0.266 g) and DIPEA (0.36 mL) in DMF (2 mL) was added. The mixture was shaken at r.t. overnight. The solution was drained and the resin was washed with DMF, DCM, MeOH, DCM and finally dried to give the title compound (0.2 g).
¹H-MAS-NMR (CD₂Cl₂): δ (ppm) 7.53 (bs, 1H); 6.84 (bm, 2H); 4.0 (bm, 2H); 3.80 (bd, 1H); 3.01 (bs) and 2.8 (bs) and 2.58 (bs) (6H); 2.35 (s, 3H); 2.04 (bs, 2H linker); 1.48 (bs, 6H linker).
¹⁹F-MAS NMR (CD₂Cl₂): δ (ppm) -116.3.

### Intermediate 7

### 4-[(3,5-Difluoro-benzyl)-methyl-carbamoyl]-3-(S)-(4-fluoro-2-methyl-phenyl)-piperazine-1-carboxylic acid, tert-butyl ester

TEA (149 µL) and a solution of triphosgene (48 mg) in anhydrous DCM (3.0 mL) were added to a solution of 1-(*tert*-butoxycarbonyl)-3-(S)-(4-fluoro-2-methyl-phenyl)-piperazine (105 mg) in anhydrous DCM (4.13 mL) previously cooled to 0°C under a nitrogen atmosphere. The reaction mixture was stirred from 0°C to r.t. for 4 h.
Then, N-methyl-3,5-difluoro-benzylamine hydrochloride (83 mg) was added. The reaction mixture was stirred at r.t. for 18 h. It was then diluted with further DCM, washed with 1N hydrochloric acid solution. The organic layer was dried, concentrated *in vacuo* and the residue was purified by flash chromatography (CH/AcOEt 6:4) to give the title compound (121 mg) as a white foam.
IR (CDCl₃, cm⁻¹): 1686 (C=O), 1649 (C=O).
¹H-NMR (CDCl₃, 50°C): δ (ppm) 7.23 (dd, 1H), 6.87 (dd, 1H), 6.82 (dt, 1H), 6.67 (m, 1H), 6.52 (m, 2H), 4.59 (dd, 1H), 4.43 (d, 1H), 4.32 (d, 1H), 3.90 (bm, 2H), 3.31 (m, 2H), 3.11 (m, 2H), 2.90 (s, 3H), 1.48 (s, 9H).
MS (FAB⁺/NBA): m/z=478[MH]⁺, 477 [M]⁺.

### Intermediate 8

### 4-(2,5-Dichloro-benzyl-carbamoyl)-3-(S)-(4-fluoro-2-methyl-phenyl)-piperazine-1-carboxylic acid, tert-butyl ester

TEA (149 µL) and a solution of triphosgene (48 mg) in anhydrous DCM (3.0 mL) were added to a solution of 1-(*tert*-butoxycarbonyl)-3-(S)-(4-fluoro-2-methyl-phenyl)-piperazine (105 mg, 0.36 mmol) in anhydrous DCM (4.13 mL) previously cooled to 0°C under a nitrogen atmosphere. The reaction mixture was stirred from 0°C to r.t. for 4 h.
Then, 2,5-dichloro-benzylamine hydrochloride (97 mg) was added. The reaction mixture was stirred at r.t. for 18 h. It was then diluted with further DCM and washed with 1N hydrochloric acid solution The organic layer was dried, concentrated *in vacuo* and the residue was purified by flash chromatography (CH/AcOEt 6:4) to give the title compound (119 mg) as a yellow foam.
IR (nujol, cm⁻¹): 3340 (NH), 1698 (C=O), 1632 (C=O).
¹H-NMR (DMSO-d₆, 70°C): δ (ppm) 7.37 (d, 1H), 7.25 (dd, 1H), 7.21 (m, 1H), 7.05 (s, 1H), 6.99 (dd, 1H), 6.92 (dt, 1H), 6.73 (t, 1H), 5.19 (t, 1H), 4.32 (dd, 1H), 4.18 (dd, 1H), 3.90 (dt, 1H), 3.73 (dd, 1H), 3.7-3.58 (m, 2H), 3.54 (m, 1H), 3.26 (m, 1H), 2.34 (s, 3H), 1.29 (s, 9H).
MS (ES/+): m/z=496 [MH]⁺.

### Intermediate 9

### 4-[(2,5-Dichloro-benzyl)-methyl-carbamoyl]-3-(S)-(4-fluoro-2-methyl-phenyl)-piperazine-1-carboxylic acid, tert-butyl ester

Sodium *t*-butoxide (69 mg) was added to a solution of intermediate **8** (119 mg) in anhydrous THF (2.5 mL) at r.t. under a nitrogen atmosphere. The solution was stirred at r.t. for 15 min then iodomethane (60 µL) was added. The reaction mixture was stirred at r.t. for 60 min and was poured in a mixture of AcOEt and saturated sodium hydrogen carbonate solution. The phases were separated and the aqueous layer was extracted twice with AcOEt. The combined organic extracts were dried, concentrated *in vacuo* and the residue was purified by flash chromatography (CH/AcOEt 6:4) to give the title compound (108 mg) as a white foam.
IR (CDCl₃, cm⁻¹): 1688 (C=O), 1659 (C=O).
¹H-NMR (CDCl₃): δ (ppm) 7.25 (m, 2H), 7.15 (dd, 1H), 6.85 (m, 3H), 4.56 (bm, 1H), 4.47 (AB, 2H), 3.81, (bm, 2H), 3.33, (m, 2H), 3.10 (m, 2H), 2.96 (s, 3H), 2.42 (s, 3H), 1.47 (s, 9H).
MS (EI/+): m/z=509 [M]⁺.

### Intermediate 10

### Polymer-bound 2-(S)-(4-fluoro-2-methyl-phenyl)-piperazine-1-carboxylic acid, (3,4-dichlorobenzyl)-methylamide

The supported intermediate 6 (50 mg) was swollen in DCM (0.25 mL) and a solution of triphosgene (24 mg) in DCM (0.25 mL) was added to the suspension. Then triethylamine (0.070 mL) was added and the reaction mixture was shaken at r.t. for 2 h. The solution was drained and the resin was washed rapidly with anhydrous DCM obtaining the supported carbamoylchloride intermediate, which was immediately swollen in DCM and treated with the 3,4-dichlorobenzylamine (0.067 mL). The reaction mixture was shaken at r.t. overnight. The solution was drained and the resin was washed with DCM, MeOH, DCM and finally dried obtaining to give the title compound (50 mg).
¹H-MAS-NMR (CD₂Cl₂): δ (ppm) 7.27 (bs, 3H); 5.0 (bs, 1H); 4.3-3.8 (bm) and 3.8-3.3 (bm) (8H); 2.56 (bm, 2H linker); 2.34 (bs, 3H); 1.48 (bs, 6H linker).

### Intermediate 11

### Polymer-bound 2-(S)-(4-fluoro-2-methyl-phenyl)-piperazine-1-carboxylic acid, (3,4-dichlorobenzyl)-methylamide

The resin intermediate 10 (16 mg) was suspended in THF (0.3ML) and NaH (80% solution in mineral oil - 7.2 mg) was added. The mixture was shaken at r.t. for 5 min then methyliodide (0.024 mL) was added. Warming up of the reaction mixture was noticed and a white precipitate formed. The reaction mixture was shaken at 60°C overnight. The solution was drained and the resin was washed with MeOH and DCM, dried to give the title compound (16 mg).
¹H-MAS-NMR (CD₂Cl₂): δ (ppm) 7.28 (bm, 3H); 4.50 (bs, 1H); 4.41-4.2 (2bd, 2H); 3.93 (broad, 2H); 3.28 (bm, 2H); 3.01 (bm, 2H); 2.85 (bs, 3H); 2.58 (bs, 2H linker); 2.41 (s, 3H); 1.48 (s, 6H linker).

### Example 1

### 2-(S)-(4-Fluoro-2-methyl-phenyl)-piperazine-1-carboxylic acid (2,4-dimethyl-benzyl)-methylamide acetate

Borane (1M solution in THF - 1.93 mL) was added to a solution of intermediate 1 (123 mg) in anhydrous THF (3.2 mL) at r.t. under a nitrogen atmosphere. The solution was heated at reflux for 4 hr. It was then cooled down to r.t. and conc. hydrochloric acid (2 mL) was added to destroy the excess of borane. The reaction mixture was stirred at 23°C for 1 h., then water was added, followed by potassium carbonate until gas evolution ceased (pH 8-9). The aqueous layer was extracted three times with AcOEt. The combined organic extracts were dried, concentrated *in vacuo* and the residue was purified by flash chromatography (AcOEt/MeOH/conc. NH₄OH 90:6:4) to give the pure 2-(S)-(4-fluoro-2-methyl-phenyl)-piperazine-1-carboxylic acid, (2,4-dimethyl-benzyl)-methylamide (95 mg).
This compound was dissolved in anhydrous Et₂O (2 mL) and AcOH (37 µL) was added. The solvent was evaporated to give the title compound (71 mg) as a yellow glassy solid.
IR (nujol, cm⁻¹): 1709 (C=O), 1661 (C=O) cm⁻¹.
¹H-NMR (DMSO-d₆): δ (ppm) 9.00-7.00 (b, 2H), 7.35 (dd, 1H), 6.92-6.85 (m, 3H), 6.83 (d, 1H), 6.67 (d, 1H), 4.36 (d, 1H), 4.22 (dd, 1H), 4.17 (d, 1H), 3.20 (m, 1H), 2.80 (s, 3H), 2.90-2.70 (m, 4H), 2.54 (m, 1H), 2.32 (s, 3H), 2.21 (s, 3H), 2.07 (s, 3H).
MS (FAB⁺/NBA): m/z= 370 [M-AcO]⁺.

### Example 2

### 2-(S)-(4-Fluoro-2-methyl-phenyl) piperazine-1-carboxylic acid, (3,5-dimethyl-benzyl)-methylamide hydrochloride

Borane (1M solution in THF - 1.8 mL) was added at 23°C to a suspension of intermediate 2 (110 mg) in anhydrous THF (5 mL), under a nitrogen atmosphere, and the reaction mixture was refluxed for 2 h. It was then cooled down to r.t. and conc. hydrochloric acid (158 µL) was added. The mixture was stirred at 23°C for 2 h, then DCM (15 mL) was added. The obtained organic phase was washed twice with a saturated solution of sodium hydrogen carbonate, dried and concentrated *in vacuo* to give 2-(S)-(4-fluoro-2-methyl-phenyl) piperazine-1-carboxylic acid, (3,5-dimethyl-benzyl)-methylamide (70 mg). This material was dissolved in Et₂O (2 mL) and treated with HCl (1M solution in Et₂O - 0.5 mL). The wet precipitate initially obtained was stirred in a mixture of Et₂O/petroleum to give the title compound (60 mg), as a white solid.
¹H-NMR (d₆-DMSO): δ (ppm) 9.36 (d, 2H); 7.36 (s, 1H); 7.01-6.96 (m, 2H); 6.82 (s, 1H); 6.47 (s, 2H); 4.54 (d, 1H); 4.37 (d, 1H); 4.16 (d, 1H); 3.62-2.88 (m, 6H); 2.81 (s, 3H); 2.39 (s, 3H); 2.14 (s, 6H).
MS (FAB⁺/NBA): m/z=370 [MH-Cl]⁺.

### Example 3

### 2-(S)-(4-Fluoro-2-methyl-phenyl)-piperazine-1-carboxylic acid, (3,5-dichloro-benzyl)-methylamide hydrochloride

Borane (1M solution in THF - 2.0 mL) was added to a solution of intermediate 3 (135 mg) in anhydrous THF (3.3 mL), under a nitrogen atmosphere at r.t.. The solution was heated at reflux for 4 h. It was then cooled down to r.t. and conc. hydrochloric acid (2 mL) was added to destroy the excess of borane. The reaction mixture was stirred at r.t. for 1 h, then water was added followed by potassium carbonate until gas evolution ceased (pH 8-9). The aqueous layer was extracted three times with AcOEt. The combined organic extracts were dried and concentrated *in vacuo* to a residue which was purified by flash chromatography (AcOEt/MeOH/conc. NH₄OH 90:6:4) to give 2-(S)-(4-fluoro-2-methyl-phenyl)-piperazine-1-carboxylic acid, (3,5-dichloro-benzyl)-methylamide (134 mg).
This compound was dissolved in anhydrous Et₂O (2 mL) and treated with HCl (1.0M in Et₂O - 662 µL). The solvent was evaporated to give the title compound (110 mg) as a yellow solid.
m.p.: 110-112°C
IR (nujol, cm⁻¹): 3376 (NH), 1653 (C=O).
¹H-NMR (DMSO-d₆): δ (ppm) 9.29 (bs, 2H), 7.38 (t, 1H), 7.36 (dd, 1H), 6.96 (dd, 1H), 6.93 (d, 2H), 6.91 (td, 1H), 4.60 (dd, 1H), 4.45 (d, 1H), 4.25 (d, 1H), 3.50 (m, 1H), 3.40 (m, 1H), 3.29 (m, 1H), 3.22 (m, 1H), 3.11 (m, 1H), 2.95 (t, 1H), 2,90 (s, 3H), 2.40 (s, 3H).
MS (FAB⁺/NBA): m/z=410 [M]⁺.

### Example 4

### 2-(S)-(4-Fluoro-2-methyl-phenyl)-piperazine-1-carboxylic acid, (3-chloro-4-fluorobenzyl)-methylamide hydrochloride

Palladium on charcoal (10% - 33 mg) was added to a solution of intermediate 4 (0.130 g) in anhydrous MeOH (5 mL), at r.t. under a nitrogen atmosphere. The reaction mixture was placed under hydrogen atmosphere and stirred at P and T atm. for 4 h. The catalyst was then filtered off and rinsed with AcOEt. The solution was concentrated *in vacuo* to give 2-(S)-(4-fluoro-2-methyl-phenyl)-piperazine-1-carboxylic acid, (2-chloro-3-fluoro-benzyl)-methylamide (112 mg).
This material was dissolved in anhydrous Et₂O (2 mL) and treated with HCl (1M solution in Et₂O - 492 µL). The solid precipitated was isolated by filtration to give the title compound (84 mg) as a yellow solid.
¹H-NMR (DMSO-d₆): δ (ppm) 9.05 (bs, 2H), 7.35 (dd, 1H), 7.22 (t, 1H), 7.05-6.85 (m, 4H), 4.57 (dd, 1H), 4.40 (dd, 1H), 4,25 (dd, 1H), 3.48 (m, 1H), 3.39 (m, 1H), 3.27 (m, 2H), 2.98 (m, 2H), 2.90 (s, 3H), 2.41 (s, 3H).
MS (ES/+): m/z= 394 [M-Cl]⁺.

### Example 5

### 2-(S)-(4-Fluoro-2-methyl-phenyl)-piperazine-1-carboxylic acid, (4-fluoro-benzyl)-methylamide hydrochloride

Palladium over charcoal (10% - 15 mg) was added to a solution of intermediate 5 (0.61 g) in anhydrous MeOH (2.5 mL) at r.t. under a nitrogen atmosphere. The reaction mixture was placed under hydrogen atmosphere and stirred at P and T atm. for 4 h. The catalyst was then filtered and rinsed with AcOEt. The solution was concentrated *in vacuo* to give 2-(S)-(4-fluoro-2-methyl-phenyl)-piperazine-1-carboxylic acid, (4-fluoro-benzyl)-methylamide (64 mg).
This material was dissolved in anhydrous Et₂O (2 mL) and treated with HCl (1M solution in Et₂O - 248 µL). The solid formed was filtered, rinsed with Et₂O and dried to give the title compound hydrochloride (17 mg) as a white solid.
m.p.: 108-110°C
¹H-NMR (DMSO-d₆): δ (ppm) 9.22 (bs, 2H), 7.40 (dd, 1H), 7.04-6.99 (m, 6H), 4.58 (dd, 1H), 4.34 (m, 2H), 3.44 (d, 1H), 3.37 (d, 1H), 3.29 (dd, 1H), 3.22 (dd, 1H), 3.10 (m, 1H), 2.96 (t, 1H), 2.84 (s, 3H), 2.40 (s, 3H).

### Example 6

### 2-(S)-(4-Fluoro-2-methyl-phenyl)-piperazine-1-carboxylic acid, (3,5-difluoro-benzyl)-methylamide hydrochloride

Trifluoroacetic acid (1.75 mL) was added to a solution of intermediate 7 (165 mg) in anhydrous DCM (1.75 mL), at r.t. under a nitrogen atmosphere. The solution was stirred at r.t. for 15 min. then it was poured in 1N sodium hydroxide solution and the phases were separated. The aqueous phase was extracted twice with DCM. The combined organic extracts were dried and concentrated *in vacuo* to give 2-(S)-(4-fluoro-2-methyl-phenyl)-piperazine-1-carboxylic acid, (3,5-difluoro-benzyl)-methylamide (123 mg). This material was dissolved in anhydrous Et₂O (2 mL) and treated with HCl (1M solution in Et₂O - 519 µL). The solid formed was filtered, rinsed with Et₂O and dried to give the title compound (94 mg) as a white solid.
IR (nujol, cm⁻¹): 3388 (NH₂⁺), 1659 (C=O), 1621 (C=C).
¹H-NMR (DMSO-d₆): δ (ppm) 9.19 (bs, 1H), 8.96 (bs, 1H), 7.36 (dd, 1H), 7.06 (tt, 1H), 7.03 (dd, 1H), 6.93 (td, 1H), 6.51 (bd, 2H), 4.49 (dd, 1H), 4.46 (d, 1H), 4.19 (d, 1H), 3.47 (d, 1H), 3.38 + 3.30 (m, 1H), 3.26 (m, 1H), 3.01 (m, 1H), 2.93 (m, 1H), 2.91 (s, 3H), 2.38 (s, 3H).
MS (FAB⁺/NBA): m/z= 378 [MH]⁺.

### Example 7

### 2-(S)-(4-Fluoro-2-methyl-phenyl)-piperazine-1-carboxylic acid, (2,5-dichloro-benzyl)-methylamide hydrochloride

Trifluoroacetic acid (2.0 mL) was added to a solution of intermediate 9 (103 mg) in anhydrous DCM (2.0 mL) at r.t. under a nitrogen atmosphere. The solution was stirred at r.t. for 30 min., then it was poured in 1N sodium hydroxide solution and the phases were separated. The aqueous phase was extracted twice with DCM. The combined organic extracts were dried and concentrated *in vacuo* to give 2-(S)-(4-fluoro-2-methyl-phenyl)-piperazine-1-carboxylic acid, (2,5-dichloro-benzyl)-methylamide (106 mg). This material was dissolved in anhydrous Et₂O and treated with HCl (1 M solution in Et₂O - 519 µL). A solid formed was filtered, rinsed with Et₂O and dried to give the title compound (81 mg) as a white solid.
IR (nujol, cm⁻¹): 3428 (NH₂⁺), 1666 (C=O).
¹H-NMR (acetone-d₆): δ (ppm) 10.51 (bs, 2H), 7.55 (dd, 1H), 7.40 (d, 1H), 7.28 (dd, 1H), 6.93 (m, 2H), 6.78 (d, 1H), 4.98 (dd, 1H), 4.54 (d, 1H), 4.45 (d, 1H), 3.72 (dd, 1H), 3.67 (m, 1H), 3.48 (m, 2H), 3.38 (dd, 1H), 3.12 (m, 1H), 2.82 (s, 3H), 2.48 (s, 3H).
MS (ES/+): m/z= 410 [M-Cl]⁺.

### Example 8

### 2-(S)-(4-fluoro-2-methyl-phenyl)-piperazine-1-carboxylic acid, (3,4-dichlorobenzyl)-methylamide trifluoroacetate

The resin intermediate 11 (11 mg) was suspended in a 10% trifluoroacetic acid solution in DCM (0.5 mL) and shaken at rt for 1.5h. The resin was filtered and washed with DCM. The solutions were collected and concentrated *in vacuo* to give the title compound (4.0 mg) as a yellow wax.
¹H-NMR (CDCl₃): δ (ppm) 7.29 (d, 1H); 7.17 (dd, 1H); 6.97 (d, 1H); 6.92-6.84 (m, 2H); 6.71 (dd, 1H), 4.7 (dd, 1H); 4.55 (d, 1H); 4.15 (d, 1H); 3.53 (m, 1H), 3.43 (m, 2H); 3.33 (m, 2H); 3.06 (t, 1H); 2.94 (s, 3H); 2.42 (s, 3H).

### Pharmacy Examples

### A. Capsules/ Tablets

| | |
|---|---|
| Active ingredient | 20.0mg |
| Starch 1500 | 2.5mg |
| Microcrystalline Cellulose | 200.0mg |
| Croscarmellose Sodium | 6.0mg |
| Magnesium Stearate | 1.5mg |

The active ingredient is blended with the other excipients. The blend can be used to fill gelatin capsules or compressed to form tablets using appropriate punches. The tablets can be coated using conventional techniques and coatings.

### B. Tablets

| | |
|---|---|
| Active ingredient | 20.0mg |
| Lactose | 200.0mg |
| Microcrystalline | 70.0mg |
| Cellulose | |
| Povidone | 25.0mg |
| Croscarmellose Sodium | 6.0mg |
| Magnesium Stearate | 1.5mg |

The active ingredient is blended with lactose, microcrystalline cellulose and part of the croscannellose sodium. The blend is granulated with povidone after dispersing in a suitable solvent (i.e. water). The granule, after drying and comminution is blended with the remaining excipients. The blend can be compressed using appropriate punches and the tablets coated using conventional techniques and coatings.

### C. Bolus

| | |
|---|---|
| Active ingredient | 2-60 mg/ml |
| Sodium phosphate | 1.0-50.0mg/ml |
| water for injection | qs to 1ml |

The formulation may be packed in glass ampoules or vials and syringes with a rubber stopper and a plastic/metal overseal (vials only).

### D. Infusion

| | |
|---|---|
| Active ingredient | 2-60 mg/ml |
| Infusion solution (NaCl 0.9% or 5% dextrose) | qs to 100ml |

The formulation may be packed in glass vials or plastic bag.

The affinity of the compound of the invention for NK1 receptor was determined using the NK₁-receptor binding affinity method measuring in vitro by the compounds' ability to displace [3H] - substance P (SP) from recombinant human NK₁ receptors expressed in Chinese Hamster Ovary (CHO) cell membranes. The affinity values are expressed as negative logarithm of the inhibition constant (Ki) of displacer ligands (pKi).

The pKi values obtained as the average of at least two determinations with representative compounds of the invention are given in the following table:

| Example No | pki |
|---|---|
| 2 | 9.14 |
| 3 | 9.43 |
| 6 | 8.78 |
| 7 | 9.07 |
| 8 | 9.19 |

## Claims

1. A compound of formula (I) 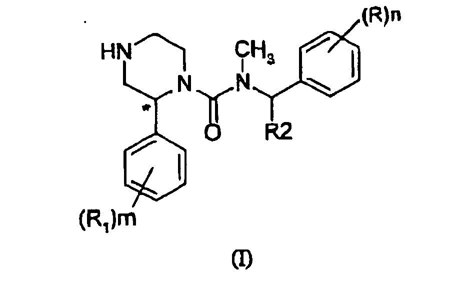 wherein
R and R₁ represent independently halogen or C₁₋₄ alkyl;
R₂ is hydrogen or C₁₋₄ alkyl;
n and m are independently 1 or 2
and pharmaceutically acceptable salts and solvates thereof.

2. A compound as claimed in claim 1 wherein the chiral carbon atom indicated as * is in β configuration.

3. A compound as claimed in claim 1 or claim 2 wherein m is 2 and R₁ is at the 2 and the 4 position of the phenyl ring.

4. A compound as claimed in any claims 1 to 3 wherein R is selected from fluorine, chlorine or methyl, R₁ is fluorine or methyl at the 2 and the 4 position of the phenyl ring, R₂ is hydrogen or methyl, n and m are 2.

5. 2-(S)-(4-Fluoro-2-methyl-phenyl)-piperazine-1-carboxylic acid, (2,5-dichlorobenzyl)-methylamide and pharmaceutically acceptable salts and solvates thereof.

6. A compound selected from:
2-(S)-(4-Fluoro-2-methyl-phenyl)-piperazine-1-carboxylic acid, (2,4-dimethyl-benzyl)-methylamide acetate;
2-(S)-(4-Fluoro-2-methyl-phenyl) piperazine-1-carboxylic acid, (3,5-dimethyl-benzyl)-methylamide;
2-(S)-(4-Fluoro-2-methyl-phenyl)-piperazine-1-carboxylic acid, (3,5-dichloro-benzyl)-methylamide;
2-(S)-(4-Fluoro-2-methyl-phenyl)-piperazine-1-carboxylic acid, (3-chloro-4-fluoro-benzyl)-methylamide;
2-(S)-(4-Fluoro-2-methyl-phenyl)-piperazine-1-carboxylic acid, (4-fluoro-benzyl)-methylamide;
2-(S)-(4-Fluoro-2-methyl-phenyl)-piperazine-1-carboxylic acid, (3,5-difluoro-benzyl)-methylamide;
2-(S)-(4-fluoro-2-methyl-phenyl)-piperazine-1-carboxylic acid, (2,5-dichloro-benzyl)-methylamide;
2-(S)-(4-fluoro-2-methyl-phenyl)-piperazine-1-carboxylic acid, (3,4-dichlorobenzyl)-methylamide;
and pharmaceutically acceptable salts or solvates thereof.

7. A compound as claimed in any claims from 1 to 6 for use in therapy.

8. The use of a compound as claimed in any claims from 1 to 6 in the preparation of a medicament for use in the treatment of CNS disorders and psychotic disorders.

9. A pharmaceutical composition comprising a compound as claimed in any claims from 1 to 6 in a mixture with one or more pharmaceutically acceptable carriers or excipients.

10. A process (A) for the preparation of a compound as claimed in any claims from 1 to 6, which comprises preparation by reduction of a ketopiperazine of formula (II), wherein Rₐ is hydrogen or a suitable nitrogen protecting group 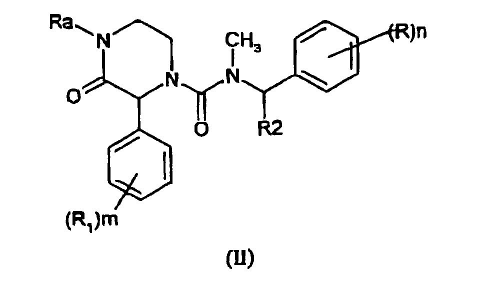 with a suitable metal reducing agent;
or a process (B) for the preparation of a compound of formula (I) as claimed in claim 1 which comprises the reaction of a compound of formula (VIII), 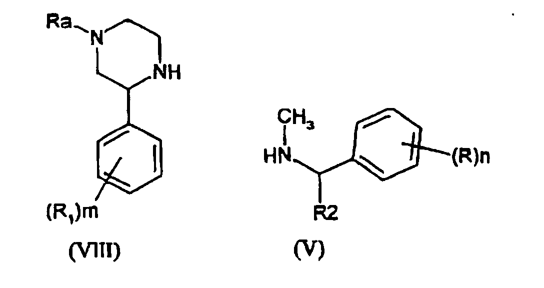 wherein Rₐ represents a nitrogen protecting group, with triphosgene and an organic base followed by addition of the amine (V);
followed where necessary or desired by one or more of the following steps:
i) removal of the nitrogen protecting group;
ii) isolation of the compound as a salt or a solvate thereof;
iii) separation of a compound of formula (I) or derivative thereof into the enantiomers thereof.

## Patentansprüche

1. Verbindung der Formel (I): 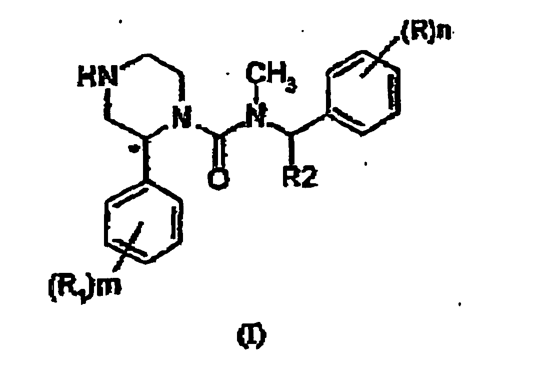 wobei
R und R₁ unabhängig Halogen oder C₁₋₄-Alkyl darstellen;
R₂ für Wasserstoff oder C₁₋₄-Alkyl steht;
n und m unabhängig 1 oder 2 darstellen;
und pharmazeutisch verträgliche Salze und Solvate davon.

2. Verbindung nach Anspruch 1, wobei das chirale Kohlenstoffatom, dargestellt als *, in β-Konfiguration steht.

3. Verbindung nach Anspruch 1 oder Anspruch 2, wobei m für 2 steht und R₁ sich in 2- und 4-Position des Phenylrings befindet.

4. Verbindung nach einem der Ansprüche 1 bis 3, wobei R ausgewählt ist aus Fluor, Chlor oder Methyl, R₁ für Fluor oder Methyl in 2- oder 4-Position des Phenylrings steht, R₂ für Wasserstoff oder Methyl steht, n und m für 2 stehen.

5. 2-(S)-(4-Fluor-2-methylphenyl)-piperazin-1-carbonsäure-(2,5-Dichlorbenzyl)-methylamid und pharmazeutisch verträgliche Salze und Solvate davon.

6. Verbindung ausgewählt aus:
2-(S)-(4-Fluor-2-methylphenyl)-piperazin-1-carbonsäure-(2,4-Dimethylbenzyl)-methylamidacetat;
2-(S)-(4-Fluor-2-methylphenyl)-piperazin-1-carbonsäure-(3,5-Dimethylbenzyl)-methylamid;
2-(S)-(4-Fluor-2-methylphenyl)-piperazin-1-carbonsäure-(3,5-Dichlorbenzyl)-methylamid;
2-(S)-(4-Fluor-2-methylphenyl)-piperazin-1-carbonsäure-(3-Chlor-4-fluorbenzyl)-methylamid;
2-(S)-(4-Fluor-2-methylphenyl)-piperazin-1-carbonsäure-(4-Fluorbenzyl)-methylamid;
2-(S)-(4-Fluor-2-methylphenyl)-piperazin-1-carbonsäure-(3,5-Difluorbenzyl)-methylamid;
2-(S)-(4-Fluor-2-methylphenyl)-piperazin-1-carbonsäure-(2,5-Dichlorbenzyl)-methylamid;
2-(S)-(4-Fluor-2-methylphenyl)-piperazin-1-carbonsäure-(3,4-Dichlorbenzyl)-methylamid;
und pharmazeutisch verträglichen Salzen und Solvaten davon.

7. Verbindung nach einem der Ansprüche 1 bis 6 zur Verwendung in der Therapie.

8. Verwendung einer Verbindung nach einem der Ansprüche 1 bis 6 bei der Herstellung eines Medikaments zur Verwendung bei der Behandlung von Störungen des zentralen Nervensystems und psychotischen Störungen.

9. Atzneimittel, umfassend eine Verbindung nach einem der Ansprüche 1 bis 6 in einem Gemisch mit einem oder mehreren pharmazeutisch verträglichen Trägern oder Hilfsstoffen.

10. Verfahren (A) zur Herstellung einer Verbindung nach einem der Ansprüche 1 bis 6, umfassend die Herstellung durch Reduktion eines Ketopiperazins der Formel (II), wobei Rₐ für Wasserstoff oder eine geeignete Stickstoffschutzgruppe steht 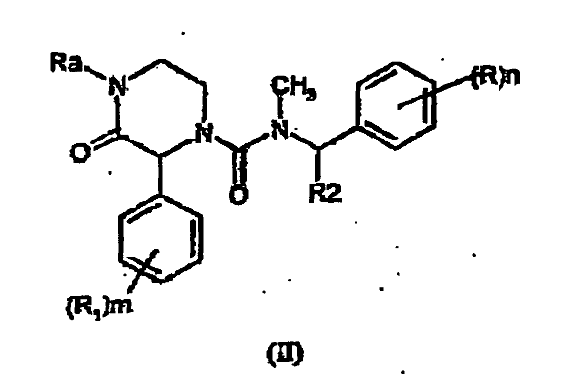 mit einem geeigneten Metall-Reduktionsmittel;
oder ein Verfahren (B) zur Herstellung einer Verbindung der Formel (I) nach Anspruch 1, umfassend die Umsetzung einer Verbindung der Formel (VIII) 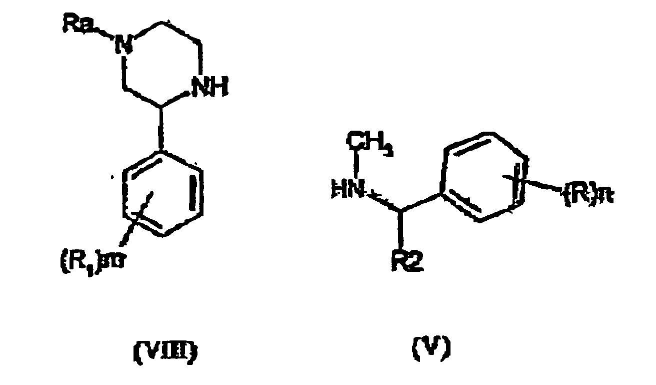 wobei Rₐ für eine Stickstoffschutzgruppe steht, mit Triphosgen und einer organischen Base, gefolgt von der Addition des Amins (V);
gefolgt, wo benötigt oder gewünscht, von einem oder mehreren der folgenden Schritte:
i) Entfernen der Stickstoffschutzgruppe;
ii) Isolieren der Verbindung als Salz oder Solvat davon;
iii) Trennen einer Verbindung von Formel (I) oder eines Derivats davon in ihre Enantiomere.

## Revendications

1. Composé de formule (I) 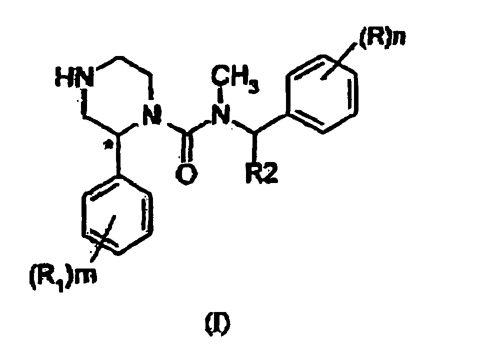 dans laquelle
R et R₁ représentent indépendamment un atome d'halogène ou un groupe alkyle en C₁ à C₄ ;
R₂ représente un atome d'hydrogène ou un groupe alkyle en C₁ à C₄ ;
n et m sont égaux indépendamment à 1 ou 2,
et ses sels et produits de solvatation pharmaceutiquement acceptables.

2. Composé suivant la revendication 1, dans lequel l'atome de carbone chiral indiqué par * est en configuration β.

3. Composé suivant la revendication 1 ou la revendication 2, dans lequel m est égal à 2 et R₁ est en position 2 et en position 4 du noyau phényle.

4. Composé suivant l'une quelconque des revendications 1 à 3, dans lequel R est choisi entre un atome de fluor, un atome de chlore et un groupe méthyle, R₁ représente un atome de fluor ou un groupe méthyle en position 2 et en position 4 du noyau phényle, R₂ représente un atome d'hydrogène ou un groupe méthyle, n et m sont égaux à 2.

5. (2,5-dichlorobenzyl)-méthylamide d'acide 2-(S)-(4-fluoro-2-méthylphényl)-pipérazine-1-carboxylique et ses sels et produits de solvatation pharmaceutiquement acceptables.

6. Composé choisi entre :
l'acétate de (2,4-diméthylbenzyl)-méthylamide d'acide 2-(S)-(4-fluoro-2-méthylphényl)-pipérazine-1-carboxylique ;
le (3,5-diméthylbenzyl)-méthylamide d'acide 2-(S)-(4-fluoro-2-méthylphényl)-pipérazine-1-carboxylique ;
le (3,5-dichlorobenzyl)-méthylamide d'acide 2-(S)-(4-fluoro-2-méthylphényl)-pipérazine-1-carboxylique ;
le (3-chloro-4-fluorobenzyl)-méthylamide d'acide 2-(S)-(4-fluoro-2-méthylphényl)-pipérazine-1-carboxylique ;
le (4-fluorobenzyl)-méthylamide d'acide 2-(S)-(4-fluoro-2-méthylphényl)-pipérazine-1-carboxylique ;
le (3, 5-difluorobenzyl)-méthylamide d'acide 2-(S)-(4-fluoro-2-méthylphényl)-pipérazine-1-carboxylique ;
le (2,5-dichlorobenzyl)-méthylamide d'acide 2-(S)-(4-fluoro-2-méthylphényl)-pipérazine-1-carboxylique ;
le (3,4-dichlorobenzyl)-méthylamide d'acide 2-(S)-(4-fluoro-2-méthylphényl)-pipérazine-1-carboxylique ;
et ses sels ou produits de solvatation pharmaceutiquement acceptables.

7. Composé suivant l'une quelconque des revendications 1 à 6, destiné à être utilisé en thérapie.

8. Utilisation d'un composé suivant l'une quelconque des revendications 1 à 6 dans la préparation d'un médicament destiné à être utilisé dans le traitement de troubles du système nerveux central (SNC) et de troubles psychotiques.

9. Composition pharmaceutique comprenant un composé suivant l'une quelconque des revendications 1 à 6 en mélange avec un ou plusieurs supports ou excipients pharmaceutiquement acceptables.

10. Procédé (A) pour la préparation d'un composé suivant l'une quelconque des revendications 1 à 6, qui comprend la préparation par réduction d'une cétopipérazine de formule (II), dans laquelle Rₐ représente un atome d'hydrogène ou groupe convenable de protection de l'atome d'azote 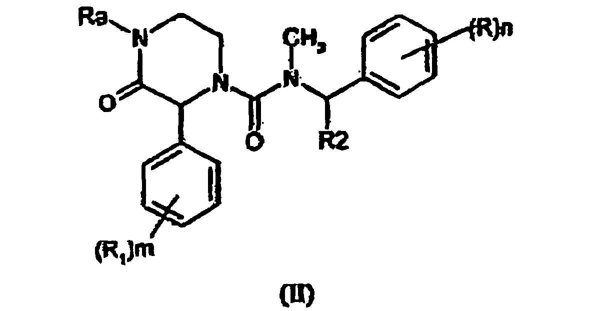 avec un agent réducteur métallique convenable ;
ou procédé (B) pour la préparation d'un composé de formule (I) suivant la revendication 1, qui comprend la réaction d'un composé de formule (VIII), 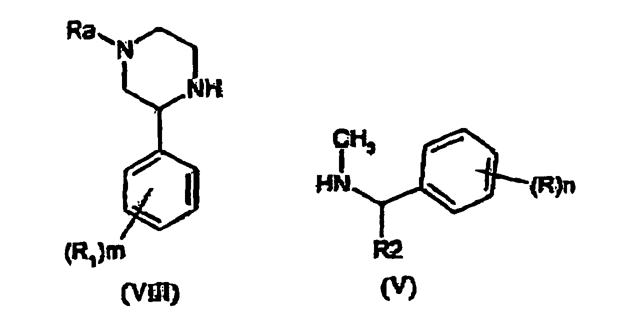 dans laquelle Rₐ représente un groupe protecteur de l'atome d'azote, avec du triphosgène et une base organique, avec ensuite l'addition de l'amine (V) ;
puis, lorsque cela est nécessaire ou désiré, une ou plusieurs des étapes suivantes :
i) élimination du groupe protecteur de l'atome d'azote ;
ii) isolement du composé sous forme d'un de ses sels ou d'un des ses produits de solvatation ;
iii) séparation d'un composé de formule (I) ou de son dérivé en ses énantiomères.
